# FASCICULE DE BREVET EUROPEEN

(11) **EP 4 028 438 B1**
(45) Date de publication et mention de la délivrance du brevet: **16.11.2022**
(21) Numéro de dépôt: 21778183.0
(22) Date de dépôt: 06.09.2021
(51) Int. Cl.: C08F 220/06, C08F 220/58, A61K 8/04, A61K 8/34, A61K 8/81, A61Q 17/00

(54) **UTILISATION DANS DES COMPOSITIONS HYDROALCOOLIQUES D'UN COPOLYMÈRE OBTENU PAR POLYMÉRISATION PAR PRÉCIPITATION**
VERWENDUNG EINES DURCH PRÄZIPITATIONSPOLYMERISATION ERHALTENEN COPOLYMERS IN WÄSSRIG-ALKOHOLISCHEN ZUSAMMENSETZUNGEN
UTILISATION DANS DES COMPOSITIONS HYDROALCOOLIQUES D'UN COPOLYMERE OBTENU PAR POLYMERISATION PAR PRECIPITATION

(30) Priorité: 07.09.2020 FR 2009044
(43) Date de publication de la demande: 20.07.2022
(73) Titulaire: SPCM SA, 42160 Andrézieux-Bouthéon (FR)
(72) Inventeur: BLONDEL, Frédéric, 42160 Andrézieux-Bouthéon (FR); MATIOSZEK, Dimitri, 42160 Andrézieux-Bouthéon (FR)
(74) Mandataire: Cabinet Laurent & Charras
(86) Numéro de dépôt international: PCT/FR2021/051521
(87) Numéro de publication internationale: WO 2022/049354

(56) Documents cités:
- EP-A1- 1 047 716
- JP-A- H09 157 130
- US-A1- 2014 086 854
- US-A1- 2020 078 287

## Description

### DOMAINE DE L'INVENTION

La présente invention concerne le domaine des compositions hydroalcooliques. Plus précisément, l'invention concerne l'utilisation, dans des compositions hydroalcooliques, d'un copolymère obtenu par polymérisation par précipitation.

### ETAT ANTERIEUR DE LA TECHNIQUE

Les virus sont à l'origine de certaines des maladies humaines les plus courantes, notamment le rhume, la varicelle et l'herpès. Certaines maladies graves, notamment celles qui sont responsables de nombreuses épidémies de la société moderne, telles que le virus Ebola, le sida, le SRAS et le SRAS-CoV-2, sont également causées par des virus.

Les virus sont des agents infectieux constitués d'un acide nucléique (ADN/ARN) dans un revêtement protéique qui s'attache aux cellules hôtes des organismes vivants et se réplique en forçant la cellule hôte à copier le matériel génétique du virus. Comme les virus peuvent se répliquer dans l'organisme malgré les mécanismes de défense de l'hôte, ils peuvent provoquer des infections chroniques à vie. En plus de leur capacité dévastatrice à provoquer des maladies graves, les virus sont également facilement transmissibles, pouvant passer par les fluides corporels, les aliments/boissons contaminés, les insectes et les contacts physiques.

Les compositions à forte teneur en alcool aident généralement à prévenir la transmission de virus et d'autres agents pathogènes par la peau car elles offrent une efficacité virucide sans irritation de la peau. Ces compositions hydroalcooliques tuent un large éventail de virus et d'autres agents pathogènes pouvant être présents sur la peau, en particulier les mains.

Des polymères épaississants sont couramment ajoutés aux compositions hydroalcooliques afin de leur donner des propriétés rhéologiques et donc de rendre leur application efficace et pratique.

Différents types de polymères formés à partir d'au moins un monomère contenant une fonction acide faible sont généralement utilisés en tant qu'agent épaississants et/ou stabilisants dans différents types d'application. On peut, par exemple, citer les brevets FR 2 810 545 et FR 2 873 126 ou encore le brevet US 3,724,547.

Le brevet EP 1 047 716 décrit des polymères à base d'acide acrylique et d'acide 2-acrylamido-2-méthylpropane sulfonique obtenus par polymérisation en émulsion inverse. Bien que ces polymères semblent avoir un effet épaississant en milieu aqueux, ils sont peu efficaces dans les compositions hydroalcooliques. En outre, ils ne permettent pas d'obtenir un gel hydroalcoolique transparent.

Le brevet JPH 09157130 décrit des polymères à base d'acide acrylique et d'acide 2-acrylamido-2-méthylpropane sulfonique obtenus par différentes techniques de polymérisation. Les polymères exemplifiés sont obtenus par une polymérisation par dispersion. Le brevet US2020/078287A1 décrit des copolymères réticulés comprenant au moins un monomère comportant un groupe acrylique et portant au moins une fonction acide faible, au moins un monomère portant au moins une fonction acide fort et un agent réticulant. Ces polymères sont obtenus par précipitation dans un solvant comprenant du tert-butanol.

Bien que les polymères de l'art antérieur soient efficaces en solution aqueuse ils ne permettent pas d'avoir des propriétés rhéologiques intéressantes en présence d'alcool et un gel hydroalcoolique transparent.

### EXPOSE DE L'INVENTION

La présente invention concerne l'utilisation, pour la fabrication d'une composition hydroalcoolique comprenant au moins de l'eau et au moins un alcool, d'un copolymère branché ou réticulé obtenu à partir de :
(i) 20 mol% à 94,99 mol% d'au moins un monomère comportant un groupe acrylique et portant au moins une fonction acide faible,
(ii) 5 mol% à 79,99 mol% d'au moins un monomère portant au moins une fonction acide fort,
(iii) 0,01 mol% à 4,0 mol% d'un agent réticulant/ramifiant, choisi parmi l'éthylène glycol di-acrylate, le polyéthylène glycol diméthacrylate, le diacrylamide, le cyanométhylacrylate, le vinyloxyéthylacrylate, le vinyloxyméthacrylate, la triallylamine, le triméthylolpropane triacrylate, le formaldéhyde, le glyoxal, les glycidyléthers, les époxy et leurs mélanges,
ledit copolymère étant obtenu par polymérisation par précipitation dans un solvant comprenant au moins un alcool.

L'invention a également pour objet l'utilisation de ce copolymère branché ou réticulé pour épaissir une composition hydroalcoolique comprenant au moins de l'eau et au moins un alcool.

L'invention concerne également les compositions hydroalcooliques comprenant de l'eau, au moins un alcool et ce copolymère branché ou réticulé.

Par « monomère portant au moins une fonction acide », on entend un monomère porteur d'une ou plusieurs fonction(s) acide libre ou neutralisée (c'est-à-dire salifiée par action d'une base). Lorsqu'un monomère comporte plus d'une fonction acide, il est possible de n'avoir qu'une partie des fonctions acide sous forme neutralisée. La ou les fonctions acides présentes peuvent être une fonction acide faible ou acide fort. En général, les monomères utilisés dans l'invention ne comportent que des fonctions acides faible ou que des fonctions acides fort, et plus préférentiellement, des monomères porteurs d'une seule fonction acide seront utilisés.

Tous les pourcentages molaires sont exprimés par rapport au nombre total de moles de monomères incluant l'agent réticulant/ramifiant. Bien entendu, l'homme du métier saura ajuster les différents pourcentages pour atteindre 100.

Le copolymère est obtenu par polymérisation par précipitation d'au moins un monomère comportant un groupe acrylique et porteur d'au moins une fonction acide faible et d'au moins un monomère comprenant au moins une fonction acide fort.

Selon l'invention, le monomère comportant un groupe acrylique et porteur d'au moins une fonction acide faible est avantageusement choisi parmi l'acide acrylique, l'acide méthacrylique, l'acide itaconique, l'acide crotonique, l'acide fumarique, l'acide maléique et leurs mélanges. Ce monomère peut être sous forme acide, partiellement salifiée ou totalement salifiée. La forme salifiée correspond préférentiellement à un sel de métal alcalin, un sel de métal alcalino-terreux ou un sel d'ammonium (NH₄⁺, ammonium primaire, secondaire, tertiaire ou quaternaire). Préférentiellement, le monomère possédant une fonction acide faible est l'acide acrylique.

Le copolymère comprend préférentiellement entre 20 et 89,99 mol% d'au moins un monomère comportant un groupe acrylique et porteur d'au moins un acide faible, plus préférentiellement entre 30 et 89,99 mol%.

De manière préférentielle 10 à 100 mol% des fonctions acides faible sont salifiées, préférentiellement 20 à 80 mol%, plus préférentiellement 30 à 70 mol%.

Le monomère portant au moins une fonction acide fort est avantageusement choisi parmi les monomères porteurs d'une fonction acide sulfonique tels que les acides acrylamidoalkylsulfoniques, par exemple l'acide 2-acrylamido-2-méthylpropane sulfonique, ou une fonction acide phosphonique. Ce monomère peut être sous forme acide, partiellement salifiée ou totalement salifiée. La forme salifiée correspond préférentiellement à un sel de métal alcalin, un sel de métal alcalino-terreux ou un sel d'ammonium (NH₄⁺, ammonium primaire, secondaire, tertiaire ou quaternaire). Préférentiellement, le monomère possédant une fonction acide fort est l'acide 2-acrylamido-2-méthylpropane sulfonique.

Selon l'invention, le copolymère comprend préférentiellement entre 10 et 79,99 mol% d'au moins un monomère porteur d'au moins un acide fort, plus préférentiellement entre 10 et 69,99 mol% par rapport à la quantité totale des monomères composant le copolymère.

De manière préférentielle, 20 à 100 mol% des fonctions acide fort sont salifiées, plus préférentiellement 40 à 100 mol% encore plus préférentiellement 60 à 100 mol%.

Sous leur forme salifiée, les fonctions acides sont sous forme anionique avec un contre-ion ou cation dépendant de la base utilisée pour la neutralisation, par exemple du type Na⁺ quand le carbonate de sodium est utilisé ou encore NH₄⁺ quand l'ammoniac (gaz) est utilisé. De manière classique, le contrôle du nombre de fonctions acide sous forme salifiée est assuré par le choix du pH de la solution de monomères qui sera ajusté en fonction du pKa des fonctions acides présentes. De préférence, il s'agit d'un sel de NH₄⁺.

De manière préférentielle, les fonctions acides sont salifiées par un agent de salification choisi parmi l'ammoniac, le carbonate de sodium, le carbonate de potassium, le carbonate d'ammonium, le bicarbonate de sodium, le bicarbonate de potassium, le bicarbonate d'ammonium, et leurs mélanges. De préférence, l'agent de salification est l'ammoniac.

De manière optionnelle, le copolymère peut comprendre au moins un monomère non ionique. Le ou les monomères non ioniques pouvant être utilisés dans le cadre de l'invention peuvent être choisis, notamment, dans le groupe comprenant les monomères vinyliques solubles dans l'eau. Des monomères préférés appartenant à cette classe sont, par exemple, l'acrylamide, le méthacrylamide, le N-isopropylacrylamide, le N,N-diméthylacrylamide et le N-méthylolacrylamide. Egalement, peuvent être utilisés la N-vinylformamide, le N-vinyl acetamide, la N-vinylpyridine et la N-vinylpyrrolidone, l'acryloyl morpholine (ACMO) et la diacétone acrylamide. Un monomère non ionique préféré est l'acrylamide.

De manière optionnelle le copolymère peut comprendre au moins un monomère cationique. Le ou les monomères cationiques pouvant être utilisés dans le cadre de l'invention peuvent être choisis, notamment parmi les monomères du type vinylique possédant une fonction ammonium quaternaire, notamment acrylamide, acrylique, allylique ou maléique possédant une fonction ammonium quaternaire. On peut citer, en particulier et de façon non limitative, l'acrylate de diméthylaminoéthyle (ADAME) quaternisé, le méthacrylate de diméthylaminoéthyle (MADAME) quaternisé, le chlorure de diméthyldiallylammonium (DADMAC), le chlorure d'acrylamido propyltriméthyl ammonium (APTAC), et le chlorure de methacrylamido propyltrimethyl ammonium (MAPTAC).

De manière préférentielle, le copolymère comprend (hors agent réticulant/ramifiant) trois monomères (terpolymère) ou moins, plus préférablement deux monomères.

De préférence, le copolymère est constitué des monomères suivants (hors agent réticulant/ramifiant) : au moins un monomère comportant un groupe acrylique et portant au moins une fonction acide faible et au moins un monomère portant au moins une fonction acide fort, lesdits monomères étant tels que décrits précédemment.

Plus préférablement, le copolymère est constitué des monomères suivants (hors agent réticulant/ramifiant) : au moins un monomère d'acide acrylique et au moins un monomère d'acide 2-acrylamido-2-méthylpropane sulfonique (ATBS).

L'agent réticulant/ramifiant est choisi parmi les agents polyfonctionnels tel que l'éthylène glycol di-acrylate, le polyéthylène glycol diméthacrylate, le diacrylamide, le cyanométhylacrylate, le vinyloxyéthylacrylate, le vinyloxyméthacrylate, la triallylamine, le triméthylolpropane triacrylate (TMPTA), le chlorure de tétraallylammonium (TAAC), le formaldéhyde, le glyoxal, les glycidyléthers comme l'éthylèneglycol diglycidyléther, les époxy et leurs mélanges. Préférentiellement, l'agent réticulant/ramifiant est le triméthylolpropane triacrylate (TMPTA).

Selon l'invention le copolymère comprend préférentiellement entre 0,01 et 2,0 mol% d'agent réticulant, préférentiellement entre 0,01 et 1,8 mol%.

La polymérisation peut être menée avec un agent de transfert, par exemple, choisi parmi le méthanol, l'alcool isopropylique, l'hypophosphite de sodium, le 2-mercaptoéthanol, le méthallysulfonate de sodium et leur mélange. Nous pouvons aussi citer les agents de transfert de type dithiocarbonate, dithiocarbamate et trithiocarbonate et leur mélange. De préférence, la quantité d'agent de transfert est comprise entre 0 et 5000 ppm en masse par rapport à la masse totale de monomères (incluant l'agent réticulant/ramifiant), et préférentiellement entre 10 et 2500 ppm.

La polymérisation par précipitation dans un milieu solvant consiste en la polymérisation de monomères solubles dans un solvant tandis que le copolymère obtenu est insoluble, et précipite donc dans le solvant. La faisabilité de ce type de polymérisation peut être assurée optionnellement par la présence d'agents tensioactifs (ou surfactants) particuliers donnant la fluidité adaptée au milieu de dispersion.

Le solvant utilisé pour la polymérisation par précipitation est préférentiellement un alcool comprenant 1 à 4 carbones. Il est avantageusement choisi parmi le méthanol, l'éthanol, le propan-1-ol, l'isopropanol, le tert-butanol, et leurs mélanges. Préférentiellement, le solvant utilisé est le tert-butanol.

Selon un mode de réalisation particulier, la polymérisation peut être réalisée en présence d'eau. Le solvant peut comprendre jusqu'à 10% en masse d'eau, préférentiellement jusqu'à 5% en masse d'eau. Dans ce cas, le solvant est constitué de 90 % à 100 % en masse d'un alcool et de 0 % à 10 % en masse d'eau.

De manière conventionnelle, la réaction de polymérisation est amorcée par introduction d'un initiateur de radicaux libres. A titre d'exemple d'agent initiateur de radicaux libres, on peut citer les couples oxydant-réducteur avec, parmi les oxydants, l'hydroperoxyde de cumène ou le butylhydroxypéroxyde tertiaire, et parmi les réducteurs, les persulfates tels que le métabisulfite de sodium et le sel de Mohr. Des composés azoïques tels que le 2,2'-azobis(isobutyronitrile), le 2,2'-azobis(2-methylbutyronitrile) et le chlorhydrate de 2,2'-azobis(2-amidinopropane) peuvent également être utilisés au même titre que des composés peroxydiques tels que le peroxyde de benzoyl, l'hydroperoxyde de tert-butyl ou le peroxyde de lauroyl.

De manière préférentielle la matière active représente 5% à 25% en masse du milieu réactionnel, plus préférentiellement 10% à 20%. La matière active comprend les monomères et l'agent réticulant/ramifiant.

Une fois la polymérisation achevée, le copolymère apparaît dans le milieu réactionnel sous la forme d'un précipité. Il s'agit généralement d'un précipité blanc. Il peut être isolé facilement en utilisant les procédés usuels de séparation (par exemple filtration ou évaporation) et de séchage. Le solvant peut être éliminé par filtration ou distillation.

Un autre aspect de l'invention concerne les compositions hydroalcooliques comprenant :
a) 55 % à 79,95 % en masse d'au moins un alcool A1,
b) 0,05 % à 5 % en masse d'au moins un copolymère branché ou réticulé comme décrit ci-dessus,
c) 15 % à 44,95 % en masse d'eau, avantageusement 20 % à 44,95 %,
d) 0 % à 10 % en masse d'au moins un additif.

Selon l'invention, l'alcool A1 compris dans la solution hydroalcoolique est avantageusement choisi parmi le méthanol, l'éthanol, l'isopropanol, le butanol, le pentanol, l'hexanol, leurs isomères et leurs mélanges. Préférentiellement, l'alcool est choisi parmi l'éthanol et/ou l'isopropanol.

Selon l'invention, la composition hydroalcoolique est avantageusement transparente. Ainsi la composition hydroalcoolique présente une transmittance avantageusement supérieure à 80 %, préférentiellement supérieure à 85 % plus préférentiellement supérieure à 90 %.

Les valeurs de transmittance peuvent être mesurées à l'aide d'un appareil de type UV-photometer DR 3900 (HACH). La transmittance correspond au pourcentage de la lumière transmise à travers un échantillon de 1 cm d'épaisseur dans une cuve en quartz, à une longueur d'onde comprise entre 400 nm et 800 nm. Elle est avantageusement mesurée en l'absence d'additif, c'est-à-dire pour une composition constituée de : a) 55 à 79,95 % en masse d'au moins un alcool A1, b) 0,05 à 5 % en masse d'au moins un copolymère branché ou réticulé comme décrit ci-dessus, et c) 15 à 44,95 % en masse d'eau.

Selon l'invention, la composition hydroalcoolique peut comprendre au moins un additif (0-10 % en masse de la composition). L'additif est avantageusement choisi parmi les tensioactifs, les agents d'ajustement du pH, les parfums, les colorants, les conservateurs, et les agents hydratant.

Préférentiellement, la composition hydroalcoolique comprend au moins un agent hydratant. L'agent hydratant peut être choisi parmi l'allantoïne ; l'acide pyrrolidonecarboxylique et les sels de celui-ci ; l'acide hyaluronique et les sels de celui-ci ; l'acide sorbique et les sels de celui-ci ; les acides aminés, par exemple l'urée, la lysine, l'arginine, la cystéine, ou la guanidine ; les polyhydroxyalcools tels que la glycérine, le propylèneglycol, l'hexylèneglycol, l'hexanetriol, l'ethoxydiglycol, le diméthiconecopolyol et le sorbitol, ainsi que les esters de ceux-ci ; le polyéthylèneglycol ; l'acide glycolique et les sels glycolate (par exemple d'ammonium et d'alkylammonium quaternaire) ; le chitosane ; les extraits d'aloe-vera ; les extraits d'algue ; le miel et les extraits de celui-ci ; l'inositol ; l'acide lactique et les sels lactate (par exemple d'ammonium et d'alkylammonium quaternaire) ; le D-panthénol ; l'ascorbylphosphate de magnésium ; l'acide kojique ; la lactamide-monoéthandamine ; l'acétamide-monoéthanolamine ; leurs analogues, et les mélanges de ceux-ci.

Préférentiellement, l'agent hydratant est la glycérine.

De manière préférentielle la composition hydroalcoolique comprend :
a) 60 % à 69,9 % en masse d'au moins un alcool,
b) 0,1 % à 3 % en masse d'au moins un copolymère branché ou réticulé comme décrit ci-dessus,
c) 25 % à 39,9 % en masse d'eau,
d) 0 % à 10% en masse d'au moins un additif.

De manière préférentielle, la composition hydroalcoolique comprend entre 0,1% et 10% en masse d'au moins un agent hydratant, de préférence de 0,5% à 8% en masse, et plus préférentiellement, de 1% à 5% en masse, par rapport à la masse totale de la composition.

L'invention et les avantages qui en découlent ressortiront mieux des exemples suivants donnés afin d'illustrer l'invention et non de manière limitative.

### Exemples de modes de réalisation de l'invention

### Protocole de préparation d'un copolymère ATBS/AA par polymérisation par précipitation

On introduit dans un réacteur à double enveloppe muni d'une pale d'agitation 460 g de tert-butanol comprenant 2,5% en masse d'eau. On ajoute, sous agitation, de l'acide 2-acrylamido-2-méthylpropane sulfonique (ATBS) et de l'acide acrylique (AA). Le milieu est bullé à l'ammoniac gazeux durant 30 minutes, tout en vérifiant que le pH ne dépasse pas 7. On dégaze la préparation dans le réacteur en injectant de l'azote durant 30 minutes. Pendant ce temps, le milieu est chauffé progressivement jusqu'à 55 °C. On ajoute ensuite le trimethylolpropane triacrylate (TMPTA, agent réticulant/ramifiant) et la température du milieu est amenée à 70 °C à l'aide d'un bain thermostaté. La réaction de polymérisation est initiée en ajoutant 0,49 g d'amorceur 2,2'-azobis(2-méthylbutyronitrile). La polymérisation démarre rapidement et atteint un maximum d'exothermie. Une fois la température maximale atteinte, le milieu est placé à reflux durant 2 heures.

On observe la présence d'un précipité blanc dans le solvant (tert-butanol + 2,5 % en masse d'eau). Ce précipité blanc correspond au copolymère de l'invention. Le solvant est ensuite éliminé à l'aide d'un évaporateur rotatif avec de l'huile chauffée à 90°C et sous une pression réduite de 100 mbars. Le copolymère obtenu est ensuite séché une nuit à 70°C.

Les copolymères A à E sont fabriqués selon le protocole ci-avant. Les polymérisations sont réalisées à 15% en masse de matière active par rapport au milieu réactionnel. Les quantités des différents monomères sont ajustées afin d'obtenir les proportions indiquées dans le tableau 2 ci-après pour chaque exemple.

**Tableau 1 : composition de la solution hydroalcoolique**

| **Composé** | **% (en masse)** |
|---|---|
| Eau | 28 |
| Polymère | 0,5 |
| Ethanol | 70 |
| Glycérine | 1,5 |

**Tableau 2 : proportion des monomères et caractéristiques dans l'eau et dans la solution hydroalcoolique**

| | **Monomères (mol%)** | | | **Caractéristiques** | | |
|---|---|---|---|---|---|---|
| | **ATBS** | **AA** | **TMPTA** | **Viscosité dans l'eau^{(a)}** | **Viscosité du gel hydroalcoolique^{(b)}** | **Transmittance du gel hydroalcoolique^{(c)}** |
| A | 67,54 | 31,79 | 0,67 | 11600 | 4500 | 95 |
| B | 56,65 | 42,73 | 0,62 | 12200 | 5400 | 95 |
| c | 29,86 | 69,68 | 0,46 | 11300 | 6100 | 95 |
| D | 9,98 | 89,8 | 0,22 | 9200 | 5500 | 95 |
| E | 5,0 | 94,84 | 0,16 | 5700 | 5100 | 95 |
| F | 56,95 | 43 | 0,05^{∗} | 35000 | <1000 | <10 |
| G | 67,95 | 32 | 0,05^{∗} | 27000 | <1000 | <10 |
| H | 25,7 | 73,8 | 0,5^{∗} | 9500 | <1000 | 50 |
| I | 50,2 | 49,78 | 0,02^{∗} | 4000 | <1000 | 70 |
| J | 84,15 | 15 | 0,85 | 13200 | 3200 | 76 |
| K | 94,05 | 5 | 0,95 | 15500 | 4100 | 64 |

| | | | | | | |
|---|---|---|---|---|---|---|
| AA : acide acrylique ATBS : acide 2-acrylamido-2-méthylpropane sulfonique TMPTA : trimethylolpropane triacrylate ^{(a)} La viscosité (cps) est mesurée à 1% en masse de copolymère dans de l'eau déionisée à 25°C, avec un viscosimètre Brookfield module RV à la vitesse de 20 tours par minute. ^{(b)} La viscosité (cps) est mesurée à 0,5% en masse de copolymère dans la solution hydroalcoolique à 25°C, avec un viscosimètre Brookfield module RV à la vitesse de 20 tours par minute. ^{(c)} La transmittance est mesurée à travers un échantillon de 1 cm d'épaisseur d'un gel hydroalcoolique selon la formule du tableau 1 pour une longueur d'onde 420 nm. ^{∗}le réticulant utilisé est le méthylènebisacrylamide (MBA) | | | | | | |

La viscosité et la transmittance des exemples A à E selon l'invention sont totalement satisfaisants. L'exemple E présente une viscosité intéressante et est transparent, mais présente toutefois un aspect gélatineux.

Les exemples A à E conformes à l'invention, montrent ainsi clairement que l'utilisation d'un polymère comprenant les monomères aux concentrations indiquées dans le tableau 2, ce polymère étant obtenu par polymérisation par précipitation dans un solvant comprenant au moins un alcool, permet d'obtenir un gel hydroalcoolique transparent, qui présente une viscosité plus élevée que les gels de l'état de l'art, rendant l'application du gel sur la peau plus simple et plus pratique.

Les contre-exemples F et G sont respectivement les exemples 1a et 1b reproduits à l'identique du brevet EP 1 047 716.

Les contre-exemples H et I sont respectivement les exemples 1 et 2 reproduits à l'identique du brevet JP 09157130.

Les contre-exemples F à K ne permettent pas d'obtenir une viscosité suffisante. De plus les gels correspondants sont partiellement opaques avec une transmittance faible pour les contre-exemples H à K, ou totalement opaques avec une transmittance inférieure à 10 pour les contre-exemples F et G.

## Revendications

1. Utilisation, pour la fabrication d'une composition hydroalcoolique comprenant au moins de l'eau et au moins un alcool, d'un copolymère branché ou réticulé obtenu à partir de :
(i) 20 mol% à 94,99 mol% d'au moins un monomère comportant un groupe acrylique et portant au moins une fonction acide faible,
(ii) 5 mol% à 79,99 mol% d'au moins un monomère portant au moins une fonction acide fort,
(iii) 0,01 mol% à 4 mol% d'un agent réticulant/ramifiant, choisi parmi l'éthylène glycol di-acrylate, le polyéthylène glycol diméthacrylate, le diacrylamide, le cyanométhylacrylate, le vinyloxyéthylacrylate, le vinyloxyméthacrylate, la triallylamine, le triméthylolpropane triacrylate, le formaldéhyde, le glyoxal, les glycidyléthers, les époxy et leurs mélanges,
ledit copolymère étant obtenu par polymérisation par précipitation, dans un solvant comprenant au moins un alcool.

2. Utilisation, pour épaissir une composition hydroalcoolique comprenant au moins de l'eau et au moins un alcool, d'un copolymère branché ou réticulé obtenu à partir de :
(i) 20 mol% à 94,99 mol% d'au moins un monomère comportant un groupe acrylique et portant au moins une fonction acide faible,
(ii) 5 mol% à 79,99 mol% d'au moins un monomère portant au moins une fonction acide fort,
(iii) 0,01 mol% à 4 mol% d'un agent réticulant/ramifiant, choisi parmi l'éthylène glycol di-acrylate, le polyéthylène glycol diméthacrylate, le diacrylamide, le cyanométhylacrylate, le vinyloxyéthylacrylate, le vinyloxyméthacrylate, la triallylamine, le triméthylolpropane triacrylate, le formaldéhyde, le glyoxal, les glycidyléthers, les époxy et leurs mélanges,
ledit copolymère étant obtenu par polymérisation par précipitation, dans un solvant comprenant au moins un alcool.

3. Utilisation selon l'une des revendications précédentes, ***caractérisée* en ce que** le monomère comportant un groupe acrylique et portant au moins une fonction acide faible est choisi parmi l'acide acrylique, l'acide méthacrylique, l'acide itaconique, l'acide crotonique, l'acide fumarique, l'acide maléique, et leurs mélanges ; ledit monomère étant sous forme acide, partiellement salifiée ou totalement salifiée.

4. Utilisation selon l'une des revendications précédentes, ***caractérisée* en ce que** 20% à 100 mol% des fonctions acide fort sont salifiées, plus préférentiellement 40% à 100 mol% encore plus préférentiellement 60 mol% à 100 mol%.

5. Utilisation selon l'une des revendications précédentes, ***caractérisée* en ce que** le monomère portant au moins une fonction acide fort est choisi parmi les acides acrylamidoalkylsulfoniques, tel que l'acide 2-acrylamido-2-méthylpropane sulfonique, ledit monomère étant sous forme acide, partiellement salifiée ou totalement salifiée.

6. Utilisation selon l'une des revendications précédentes, ***caractérisée* en ce que** 10 mol% à 100 mol% des fonctions acide faible sont salifiées, préférentiellement 20 mol% à 80 mol% plus préférentiellement 30 mol% à 70 mol%.

7. Utilisation selon l'une des revendications précédentes, ***caractérisée* en ce que** le copolymère est constitué des monomères suivants : au moins un monomère d'acide acrylique et au moins un monomère d'acide 2-acrylamido-2-méthylpropane sulfonique.

8. Utilisation selon l'une des revendications précédentes, ***caractérisée* en ce que** le solvant est constitué de 90 % à 100 % en masse d'un alcool choisi parmi le méthanol, l'éthanol, le propan-1-ol, l'isopropanol, le tert-butanol et leurs mélanges et de 0 % à 10 % en masse d'eau.

9. Utilisation selon l'une des revendications précédentes, ***caractérisée* en ce que** le copolymère branché ou réticulé est obtenu à partir de :
(i) 20 mol% à 89,99 mol% d'au moins un monomère comportant un groupe acrylique et portant au moins une fonction acide faible,
(ii) 10 mol% à 79,99 mol% d'au moins un monomère portant au moins une fonction acide fort,
(iii) 0,01 mol% à 2 mol% d'un agent réticulant/ramifiant.

10. Composition hydroalcoolique comprenant :
a) 55% à 79,95% en masse d'au moins un alcool A1,
b) 0,05% à 5% en masse copolymère branché ou réticulé obtenu à partir de :
(i) 20 mol% à 94,99 mol% d'au moins un monomère comportant un groupe acrylique et portant au moins une fonction acide faible,
(ii) 5 mol% à 79,99 mol% d'au moins un monomère portant au moins une fonction acide fort,
(iii) 0,01 mol% à 4 mol% d'un agent réticulant/ramifiant, choisi parmi l'éthylène glycol di-acrylate, le polyéthylène glycol diméthacrylate, le diacrylamide, le cyanométhylacrylate, le vinyloxyéthylacrylate, le vinyloxyméthacrylate, la triallylamine, le triméthylolpropane triacrylate, le formaldéhyde, le glyoxal, les glycidyléthers, les époxy et leurs mélanges,
c) 15% à 44,95 % en masse d'eau,
d) 0% à 10% en masse d'au moins un additif,
ledit copolymère étant obtenu par polymérisation par précipitation dans un solvant comprenant au moins un alcool.

11. Composition hydroalcoolique selon la revendication 10 , ***caractérisée* en ce que** l'alcool Al est choisi parmi le méthanol, l'éthanol, l'isopropanol, le butanol, le pentanol, l'hexanol, leurs isomères et leurs mélanges.

12. Composition hydroalcoolique selon la revendication 10 ou 11, ***caractérisée* en ce que** la composition hydroalcoolique présente une transmittance supérieure à 80% la transmittance correspondant au pourcentage de la lumière transmise à travers un échantillon de 1 cm d'épaisseur dans une cuve en quartz, à une longueur d'onde comprise entre 400 nm et 800 nm.

13. Composition hydroalcoolique selon l'une des revendications 10 à 12 , ***caractérisée* en ce que** l'additif comprend au moins un agent hydratant choisi parmi l'allantoïne ; l'acide pyrrolidonecarboxylique et les sels de celui-ci ; l'acide hyaluronique et les sels de celui-ci ; l'acide sorbique et les sels de celui-ci ; les acides aminés ; les polyhydroxyalcools tels que la glycérine, le propylèneglycol, l'hexylèneglycol, l'hexanetriol, l'ethoxydiglycol, le diméthiconecopolyol et le sorbitol, ainsi que les esters de ceux-ci ; le polyéthylèneglycol ; l'acide glycolique et les sels glycolate ; le chitosane ; les extraits d'aloe-vera ; les extraits d'algue ; le miel et les extraits de celui-ci ; l'inositol ; l'acide lactique et les sels lactate ; le D-panthénol ; l'ascorbylphosphate de magnésium ; l'acide kojique ; la lactamide-monoéthandamine ; l'acétamide-monoéthanolamine ; leurs analogues, et les mélanges de ceux-ci.

14. Composition hydroalcoolique selon l'une des revendication 10 à 13 comprenant :
a) 60% à 69,9 % en masse d'au moins un alcool,
b) 0,1% à 3 % en masse d'au moins un copolymère branché ou réticulé selon la revendication 10,
c) 25% à 39,9 % en masse d'eau.

## Patentansprüche

1. Verwendung, zur Herstellung einer wässrig-alkoholischen Zusammensetzung, die mindestens Wasser und mindestens einen Alkohol umfasst, eines verzweigten oder vernetzten Copolymers, erhalten aus:
(i) 20 Mol-% bis 94,99 Mol-% mindestens eines Monomers, das eine Acrylgruppe umfasst und mindestens eine schwache Säurefunktion trägt,
(ii) 5 Mol-% bis 79,99 Mol-% mindestens eines Monomers, das mindestens eine starke Säurefunktion trägt,
(iii) 0,01 Mol-% bis 4 Mol-% eines Vernetzungs-/ Verzweigungsmittels, ausgewählt aus Ethylenglykol-di-acrylat, Polyethylenglycol-dimethacrylat, Diacrylamid, Cyanomethylacrylat, Vinyloxyethylacrylat, Vinyloxymethacrylat, Triallylamin, Trimethylolpropan- triacrylat, Formaldehyd, Glyoxal, die Glycidylether, die Epoxy und ihre Mischungen,
dabei wird dieses Copolymer durch Präzipitationspolymerisation in einem mindestens einen Alkohol enthaltenden Lösungsmittel erhalten.

2. Verwendung, zum Verdicken einer wässrig-alkoholischen Zusammensetzung, die mindestens Wasser und mindestens einen Alkohol umfasst, eines verzweigten oder vernetzten Copolymers, erhalten aus:
(i) 20 Mol-% bis 94,99 Mol-% mindestens eines Monomers, das eine Acrylgruppe umfasst und mindestens eine schwache Säurefunktion trägt,
(ii) 5 Mol-% bis 79,99 Mol-% mindestens eines Monomers, das mindestens eine starke Säurefunktion trägt,
(iii) 0,01 Mol-% bis 4 Mol-% eines Vernetzungs-/ Verzweigungsmittels, ausgewählt aus Ethylenglykol-di-acrylat, Polyethylenglycol-dimethacrylat, Diacrylamid, Cyanomethylacrylat, Vinyloxyethylacrylat, Vinyloxymethacrylat, Triallylamin, Trimethylolpropan- triacrylat, Formaldehyd, Glyoxal, die Glycidylether, die Epoxy und ihre Mischungen,
dabei wird dieses Copolymer durch Präzipitationspolymerisation in einem mindestens einen Alkohol umfassenden Lösungsmittel erhalten.

3. Verwendung nach einem der vorangehenden Ansprüche, ***dadurch gekennzeichnet, dass*** das Monomer, das eine Acrylgruppe enthält und mindestens eine schwache Säurefunktion trägt, ausgewählt wird aus Acrylsäure, Methacrylsäure, Itaconsäure,, Crotonsäure, Fumarsäure, Maleinsäure und ihren Mischungen; wobei dieses Monomer die Form einer teilweise oder vollständig versalzten Säure hat

4. Verwendung nach einem der vorangehenden Ansprüche, ***dadurch gekennzeichnet, dass*** 20% bis 100 mol% der starken Säurefunktionen versalzen sind, besser noch 40% bis 100 mol% und noch besser 60 mol% bis 100 mol%.

5. Verwendung nach einem der vorangehenden Ansprüche, ***dadurch gekennzeichnet, dass*** das Monomer, das mindestens eine starke Säurefunktion trägt, ausgesucht wird aus Acrylamidoalkylsulfonsäuren, wie 2-acrylamid-2-methylpropan - Sulfonsäure,
wobei dieses Monomer die Form einer teilweise oder vollständig versalzten Säure hat.

6. Verwendung nach einem der vorangehenden Ansprüche, ***dadurch gekennzeichnet, dass*** 10 mol% bis 100 mol% der schwachen Säurefunktionen versalzen sind, besser noch 20 mol% bis 80 mol% und noch besser30 mol% bis 70 mol%.

7. Verwendung nach einem der vorangehenden Ansprüche, ***dadurch gekennzeichnet, dass*** das Copolymer aus den folgenden Monomeren besteht: mindestens ein Acrylsäuremonomer und mindestens ein 2-acrylamid-2-methylpropan - Sulfonsäure- Monomer.

8. Verwendung nach einem der vorangehenden Ansprüche, ***dadurch gekennzeichnet, dass*** das Lösungsmittel aus 90 % bis 100 Masse-% eines Alkohols, ausgewählt aus Methanol, Ethanol, Propan-1-ol, Isopropanol, Tert-butanol und ihren Mischungen und 0 bis 10 Masse-% Wasser besteht.

9. Verwendung nach einem der vorangehenden Ansprüche, ***dadurch gekennzeichnet, dass*** das verzweigte oder vernetzte Copolymer erhalten wird aus:
(i) 20 Mol-% bis 89,99 Mol-% mindestens eines Monomers, das eine Acrylgruppe umfasst und mindestens eine schwache Säurefunktion trägt,
(ii) 10 mol% bis 79,99 mol% mindestens eines Monomers, das mindestens eine starke Säurefunktion trägt,
(iii) 0,01 mol% bis 2 mol% eines Vernetzungs-/Verzweigungsmittels.

10. Wässrig-alkoholische Zusammensetzung, die enthält:
a) 55% bis 79,95 Masse-% mindestens eines Alkohols Al,
b) 0,05% bis 5 Masse-% eines verzweigten oder vernetzten Copolymers, das erhalten wird aus
(i) 20 mol% bis 94,99 mol% mindestens eines Monomers, das eine Acrylgruppe umfasst und mindestens eine schwache Säurefunktion trägt,
(ii) 5 Mol-% bis 79,99 Mol-% mindestens eines Monomers, das mindestens eine starke Säurefunktion trägt,
(iii) 0,01 Mol-% bis 4 Mol-% eines Vernetzungs-/Verzweigungsmittels, ausgewählt aus Ethylenglykol-di-acrylat, Polyethylenglycol-dimethacrylat, Diacrylamid, Cyanomethylacrylat, Vinyloxyethylacrylat, Vinyloxymethacrylat, Triallylamin, Trimethylolpropan- triacrylat, Formaldehyd, Glyoxal, die Glycidylether, die Epoxy und ihre Mischungen,
c) 15% bis 44,95 Masse-% Wasser,
d) 0% bis 10 Masse-% mindestens eines Zusatzes,
dabei wird dieses Copolymer durch Präzipitationspolymerisation in einem mindestens einen Alkohol umfassenden Lösungsmittel erhalten.

11. Wässrig-alkoholische Zusammensetzung nach Anspruch 10, ***dadurch gekennzeichnet, dass*** der Alkohol Al ausgewählt wird aus Ethanol, Isopropanol, Butanol, Pentanol, Hexanol und ihren Mischungen,

12. Wässrig-alkoholische Zusammensetzung nach Anspruch 10 oder 11, ***dadurch gekennzeichnet, dass*** die wässrig-alkoholische Zusammensetzung eine Transmission von über 80% aufweist, die Transmission entspricht dabei dem Prozentsatz des Lichtes, das durch ein Probestück mit einer Länge von 1cm Dicke in einem Quarzbehälter, durchgelassen wird, mit einer Wellenlänge zwischen 400 nm und 800 nm.

13. Wässrig-alkoholische Zusammensetzung nach einem der Ansprüche 10 bis 12, ***dadurch gekennzeichnet, dass*** der Zusatzstoff mindestens ein Befeuchtungsmittel enthält, ausgewählt aus Allantoin, Pyrrolidoncarboxylsäure und ihre Salze, Hyaluronsäue und ihre Salze, Sorbinsäure und ihre Salze; Aminosäuren; Polyhydroxyalkohole wie Glyzerin, Propylenglykol, Hexylenglykol, Hexantriol, Ethoxydiglycol, Dimethiconcopolyol und Sorbitol, sowie deren Ester; Polyethylenglycol; Glykolsäure und Glycolatsalze; Chitosan; Aloe-vera - Extrakte; Algen-Extrakte; Honig und seine Extrakte; Inositol ; Milchsäure und Laktatsalze; D-panthenol; Magnesium-Ascorbylphosphat; Kojisäure; Lactamid-Monoethandamin; Acetamid- Monoethanolamin; ihre Analoge und deren Mischungen.

14. Wässrig-alkoholische Zusammensetzung nach einem der Ansprüche 10 bis 13 die enthält:
a) 60% bis 69,9 Masse-% mindestens eines Alkohols;
b) 0,1% bis 3 Masse-% eines verzweigten oder vernetzten Copolymers nach Anspruch 10,
c) 25% bis 39,9 Masse-% Wasser.

## Claims

1. A use, for the manufacture of a hydroalcoholic composition comprising at least water and at least one alcohol, of a branched or cross-linked copolymer obtained from:
(i) 20 mol% to 94.99 mol% of at least one monomer containing an acrylic group and bearing at least one weak acid function,
(ii) 5 mol% to 79.99 mol% of at least one monomer bearing at least one strong acid function,
(iii) 0.01 mol% to 4 mol% of a cross-linking/branching agent selected from ethylene glycol diacrylate, polyethylene glycol dimethacrylate, diacrylamide, cyanomethylacrylate, vinyloxyethylacrylate, vinyloxymethacrylate, triallylamine, trimethylolpropane triacrylate, formaldehyde, glyoxal, glycidyl ethers, epoxies and mixtures thereof,
said copolymer being obtained by precipitation polymerization, in a solvent comprising at least one alcohol.

2. A use, for thickening a hydroalcoholic composition comprising at least water and at least one alcohol, of a branched or cross-linked copolymer obtained from:
(i) 20 mol% to 94.99 mol% of at least one monomer containing an acrylic group and bearing at least one weak acid function,
(ii) 5 mol% to 79.99 mol% of at least one monomer bearing at least one strong acid function,
(iii) 0.01 mol% to 4 mol% of a cross-linking/branching agent selected from ethylene glycol diacrylate, polyethylene glycol dimethacrylate, diacrylamide, cyanomethylacrylate, vinyloxyethylacrylate, vinyloxymethacrylate, triallylamine, trimethylolpropane triacrylate, formaldehyde, glyoxal, glycidyl ethers, epoxies and mixtures thereof,
said copolymer being obtained by precipitation polymerization, in a solvent comprising at least one alcohol.

3. The use according to one of the preceding claims, ***characterized* in that** the monomer containing an acrylic group and bearing at least one weak acid function is chosen from acrylic acid, methacrylic acid, itaconic acid, crotonic acid, fumaric acid, maleic acid, and mixtures thereof; said monomer being in acid form, partially salified, or fully salified.

4. The use according to one of the preceding claims, ***characterized* in that** 20% to 100 mol% of the strong acid functions are salified, more preferably 40% to 100 mol%, still more preferably 60 mol% to 100 mol%.

5. The use according to one of the preceding claims, ***characterized* in that** the monomer bearing at least one strong acid function is chosen from acrylamidoalkylsulphonic acids, such as 2-acrylamido-2-methylpropane sulphonic acid, said monomer being in acid form, partially salified or fully salified.

6. The use according to one of the preceding claims, ***characterized* in that** 10 mol% to 100 mol% of the weak acid functions are salified, preferably 20 mol% to 80 mol%, more preferably 30 mol% to 70 mol%.

7. The use according to one of the preceding claims, ***characterized* in that** the copolymer consists of the following monomers: at least one acrylic acid monomer and at least one 2-acrylamido-2-methylpropane sulfonic acid monomer.

8. The use according to one of the preceding claims, ***characterized* in that** the solvent consists of 90% to 100% by weight of an alcohol selected from methanol, ethanol, propan-1-ol, isopropanol, tert-butanol, and mixtures thereof and 0% to 10% by weight of water.

9. The use according to one of the preceding claims, ***characterized* in that** the branched or cross-linked copolymer is obtained from:
(i) 20 mol% to 89.99 mol% of at least one monomer containing an acrylic group and bearing at least one weak acid function,
(ii) 10 mol% to 79.99 mol% of at least one monomer bearing at least one strong acid function,
(iii) 0.01 mol% to 2 mol% of a cross-linking/branching agent.

10. A hydroalcoholic composition comprising:
a) 55% to 79.95% by weight of at least one alcohol A1,
b) 0.05% to 5% by weight of a branched or cross-linked copolymer obtained from:
(i) 20 mol% to 94.99 mol% of at least one monomer having an acrylic group and bearing at least one weak acid function,
(ii) 5 mol% to 79.99 mol% of at least one monomer bearing at least one strong acid function,
(iii) 0,01 mol% to 4 mol% of a cross-linking/branching agent selected from ethylene glycol diacrylate, polyethylene glycol dimethacrylate, diacrylamide, cyanomethylacrylate, vinyloxyethylacrylate, vinyloxymethacrylate, triallylamine, trimethylolpropane triacrylate, formaldehyde, glyoxal, glycidyl ethers, epoxies and mixtures thereof,
c) 15% to 44.95% by weight of water,
d) 0% to 10% by weight of at least one additive,
said copolymer being obtained by precipitation polymerization in a solvent comprising at least one alcohol.

11. The hydroalcoholic composition according to claim 10, ***characterized* in that** the alcohol A1 is chosen from methanol, ethanol, isopropanol, butanol, pentanol, hexanol, the isomers, and mixtures thereof.

12. The hydroalcoholic composition of claim 10 or 11, ***characterized* in that** the hydroalcoholic composition has a transmittance greater than 80% the transmittance corresponding to the percentage of light transmitted through a 1 cm thick sample in a quartz cell, at a wavelength between 400 nm and 800 nm.

13. The hydroalcoholic composition according to one of claims 10 to 12, ***characterized* in that** the additive comprises at least one moisturizing agent selected from allantoin; pyrrolidonecarboxylic acid and salts thereof; hyaluronic acid and salts thereof; sorbic acid and salts thereof; amino acids; polyhydroxy alcohols such as glycerin, propylene glycol, hexylene glycol, hexanetriol, ethoxydiglycol, dimethiconecopolyol, and sorbitol, as well as esters thereof; polyethylene glycol; glycolic acid and glycolate salts; chitosan; aloe vera extracts; algal extracts; honey and extracts thereof; inositol; lactic acid and lactate salts; D-panthenol; magnesium ascorbylphosphate; kojic acid; lactamide-monoethandamine; acetamide-monoethanolamine; their analogs, and mixtures thereof.

14. A hydroalcoholic composition according to any of claims 10 to 13, comprising:
a) 60% to 69.9% by weight of at least one alcohol,
b) 0.1% to 3% by weight of at least one branched or cross-linked copolymer according to claim 10,
c) 25% to 39.9% by weight of water.
